# EUROPÄISCHE PATENTSCHRIFT

(11) **EP 1 746 938 B1**
(45) Veröffentlichungstag und Bekanntmachung des Hinweises auf die Patenterteilung: **25.03.2015**
(21) Anmeldenummer: 05761585.8
(22) Anmeldetag: 14.05.2005
(51) Int. Cl.: A61B 5/15

(54) **VORRICHTUNG UND VERFAHREN ZUR POSITIONIERUNG EINES KÖRPERTEILS**
DEVICE AND METHOD FOR POSITIONING A BODY PART
DISPOSITIF ET PROCEDE POUR POSITIONNER UNE PARTIE DE CORPS

(30) Priorität: 21.05.2004 DE 102004024970
(43) Veröffentlichungstag der Anmeldung: 31.01.2007
(73) Patentinhaber: F.Hoffmann-La Roche AG, 4070 Basel (CH); Roche Diagnostics GmbH, 68305 Mannheim (DE)
(72) Erfinder: DECK, Frank, 67150 Niederkirchen (DE)
(74) Vertreter: Pfiz, Thomas
(86) Internationale Anmeldenummer: PCT/EP2005/005301
(87) Internationale Veröffentlichungsnummer: WO 2005/112763

(56) Entgegenhaltungen:
- WO-A-01/89383
- WO-A-98/24366
- WO-A-02/100276
- US-A- 6 093 156

## Beschreibung

Die Erfindung betrifft eine Vorrichtung und ein Verfahren zur Positionierung eines Körperteils, insbesondere einer Fingerbeere, an einem Analysegerät für Körperflüssigkeiten sowie ein entsprechendes Analysegerät gemäß dem Oberbegriff der unabhängigen Patentansprüche.

Aus der DE 100 26 172 A1 ist ein System zur Entnahme von Körperflüssigkeit bekannt, bei dem ein deformierbarer Doppelkonus als Kompressionseinheit für eine Erhöhung des Innendrucks in einem Bereich des angedrückten Körperteils sorgt. Dabei wird der Andruck teilweise in eine Bewegung in eine Sekundärrichtung mit Anteil quer zur Primärrichtung umgewandelt, bis der obere und untere Konusbereich aneinander anliegen. In diesem Zustand befindet sich die für eine Abschnürung des Körperteils sorgende Engstelle oberhalb des plattenförmigen Trägerteils, während die Einstich- bzw. Blutentnahmestelle noch innerhalb des umgeklappten Doppelkonus liegt. Dadurch ist ein punktförmiger Zugriff für die Blutentnahme erforderlich, wobei die Gefahr besteht, dass überschüssiges Blut die Vorrichtung verunreinigt. Zudem kann das Körperteil ungewollt Verrutschen, wenn der Reibungskoeffizient zwischen Kompressionseinheit und Haut beispielsweise aufgrund von Hautschweiß oder Fettablagerungen eine untere Grenze unterschreitet. In diesem Fall kann auch eine Fehlfunktion eintreten, wenn die Kompressionseinheit komplett in das Geräteinnere durchklappt.

Ausgehend hiervon liegt der Erfindung die Aufgabe zugrunde, die im Stand der Technik aufgetretenen Nachteile zu vermeiden und eine Positioniervorrichtung und ein entsprechendes Verfahren dahingehend zu verbessern, dass eine zuverlässige definierte Positionierung eines Körperteils frei von Störkonturen für eine insbesondere flächige Flüssigkeitsabnahme mit einfachen Mitteln erreicht wird.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, eine möglichst große Eindrücktiefe für das Körperteil in das Geräteinnere hinein sicher zu stellen. Dementsprechend wird erfindungsgemäß vorgeschlagen, dass das Kompressionselement eine in das Gehäuse eindrückbare Eingriffspartie für das Körperteil aufweist, so dass ein Entnahmebereich des Körperteils im Inneren des Gehäuses gegenüber einem flächigen Testfeld zur Flüssigkeitsentnahme exponiert ist. Auf diese Weise kann der Freiraum im Geräteinneren für einen Flüssigkeitsauftrag auf ein flächiges Substrat genutzt werden, ohne dass ein Flüssigkeitstransport beispielsweise über eine Kapillarstruktur erforderlich wäre. Die Entnahme unterhalb von Gehäusekanten erlaubt einen hygienischen Messablauf mit geringen Flüssigkeitsmengen (Blut oder Gewebeflüssigkeit), während die tief liegende Eingriffspartie für eine ortsfeste Positionierung sorgt. Zugleich ist durch die flächige Aufnahme auf dem Testfeld eine hohe Positionstoleranz gegeben.

Vorteilhafterweise ist die Eingriffspartie zumindest bis zu der Ebene einer gehäuseseitigen Begrenzung des Trägerteils eindrückbar, so dass der überstehende bzw. abgeschnürte Bereich des Körperteils frei zugänglich liegt.

Für eine weitere Verbesserung der Positioniergenauigkeit sind Anschlagmittel zur Begrenzung der Eindrücktiefe des Kompressionselements vorgesehen.

Ein besonderer Aspekt der Erfindung liegt darin, dass das im Querschnitt ringförmige Kompressionselement an einer freien Außenkante durch eine nach außen abstehende Anschlagwulst verbreitert ist. Dadurch wird beim Eindrücken eine Anschlagsituation erreicht, in der ein weiteres Durchstülpen des Kompressionselements zuverlässig verhindert wird.

Eine vorteilhafte Ausgestaltung sieht vor, dass das Kompressionselement zwei in Richtung der Andrückachse konisch aufeinander zulaufende Teilbereiche aufweist, wobei der distale Teilbereich eine beim Andrücken des Körperteils außenseitig gegen den proximalen Teilbereich anschlagende Anschlagwulst aufweist. Um ein sicheres Abstoppen zu gewährleisten, ist es von Vorteil, wenn die Anschlagwulst sich von der freien Außenkante des Kompressionselements weg vorzugsweise keilförmig verjüngt.

Alternativ oder ergänzend ist es auch möglich, dass das Trägerteil eine Öffnung zum Eindrücken des Kompressionselements aufweist, und dass gehäuseseitig an dem Trägerteil ein in den Öffnungsbereich ragendes Widerlager zur Begrenzung der Eindrücktiefe des Kompressionselements angebracht ist. Für eine sichere Abstützung ist es günstig, wenn das Widerlager durch einen in die Öffnung des Trägerteils hinein sich verjüngenden Anschlagring gebildet ist.

Für einen möglichst tief reichenden Eingriff ist es von Vorteil, wenn das Kompressionselement mit einem Öffnungsrand des Trägerteils fest verbunden ist, wobei die Verbindungsfläche sich über die gesamte Höhe des Öffnungsrandes erstreckt, so dass das Einstülpen bei der Kompression möglichst tief im Gehäusebereich erfolgt.

Um die Krafteinleitung zu optimieren, ist es vorteilhaft, wenn die Verbindungsfläche zwischen Trägerteil und Kompressionselement als Schräge von einer gehäuseseitigen Begrenzungsfläche des Trägerteils abgewandt ist, wobei der Winkel, zwischen der Schräge und der Begrenzungsfläche im Bereich von 50° bis 70°, vorzugsweise bei 60° liegen sollte.

Für den Kompressionsvorgang ist es auch vorteilhaft, wenn das Kompressionselement im Ausgangszustand eine auf eine gehäuseseitigen Randkante des Trägerteils knickfrei zulaufende Innenfläche aufweist.

Zur verbesserten Anpassung an eine gewölbte Körperteilkontur und zur sicheren Haftung ist es vorteilhaft, wenn die Eingriffspartie durch die Innenkante einer konkav gewölbten Ringfläche des Kompressionselements gebildet ist.

Herstellungstechnisch ist es günstig, wenn das Kompressionselement als Formteil, vorzugsweise als Spritzgussteil an dem vorgefertigten Trägerteil angeformt ist. Dabei kann das Kompressionselement über eine insbesondere durch einen Primer gebildete Klebeverbindung an dem Trägerteil befestigt werden.

Vorteilhafterweise besteht das Kompressionselement aus Silikon, Gummi oder Polyurethan. Aus hygienischen Gründen wird bevorzugt Silikon eingesetzt.

In baulich vorteilhafter Ausführung ist das Trägerteil als formstabile Ringscheibe insbesondere aus Metall ausgebildet.

Eine einstufige Herstellung lässt sich dadurch realisieren, dass das aus einem thermoplastischen Elastomer bestehende Kompressionselement und das aus Kunststoff bestehende Trägerteil als Zweikomponenten-Spritzgussteil ausgebildet sind.

Um einen Austausch beispielsweise zur Anpassung an die Körperteilgröße oder zur Reinigung zu erlauben, ist das Trägerteil lösbar in eine Gehäuseaufnahme einsetzbar.

Für eine kompakte Bauform eines Handgeräts ist es günstig, wenn die Gesamtbreite des Trägerteils senkrecht zur Andrückrichtung weniger als 25 mm, vorzugsweise weniger als 20 mm beträgt.

Ein weiterer Aspekt der Erfindung betrifft ein Analysegerät für Körperflüssigkeiten, insbesondere zur Blutzuckerbestimmung, mit einem Gehäuse und einer Vorrichtung zur Positionierung eines Körperteils, insbesondere einer Fingerbeere, umfassend ein vorzugsweise ringförmiges flexibles Kompressionselement zum Andrücken des Körperteils unter Druckerhöhung und ein starres Trägerteil zur Halterung des Kompressionselements an dem Gehäuse,. Hier wird erfindungsgemäß vorgeschlagen, dass das Kompressionselement eine in das Gehäuse eindrückbare Eingriffspartie für das Körperteil aufweist, so dass ein Entnahmebereich des Körperteils im Inneren des Gehäuses gegenüber einem flächigen Testfeld zur Flüssigkeitsentnahme exponiert ist. Damit können die bereits zuvor beschriebenen Vorteile in einem kompakten Handgerät realisiert werden.

Zur Magazinierung ist bevorzugt ein Testband im Gehäuseinneren angeordnet sein, welches auf einem aufwickelbaren Trägerband eine Vielzahl von Testfeldern aufweist. Weiterhin sind für ein integriertes System im Gehäuseinneren eine Stecheinrichtung zum Einstechen in den Entnahmebereich des Körperteils sowie eine Analyseeinrichtung zum Nachweis eines Analyten auf einem mit Körperflüssigkeit beaufschlagten Testfeld angeordnet.

Vorteilhafterweise sind die aufeinanderfolgend auf dem vorzugsweise in einer Kassette befindlichen Testband angeordneten Testfelder durch eine Bandtransportvorrichtung sukzessive in eine aktive Position bezüglich des Entnahmebereichs des Körperteils, der Stecheinrichtung und/oder der Analyseeinrichtung bewegbar.

Eine weitere Vereinfachung wird dadurch erreicht, dass die Stechvorrichtung eine distale Andrückfläche zum Andrücken eines Testfelds gegen den Entnahmebereich des Körperteils aufweist.

Um die Flüssigkeitsaufnahme gerade auch bei kleinen Mengen möglichst positionstolerant zu gestalten, ist es von besonderem Vorteil, wenn die mit Körperflüssigkeit beaufschlagbare Aufnahmefläche des Testfelds mehr als 2x5 mm², vorzugsweise etwa 5 x 20 mm² beträgt.

In verfahrensmäßiger Hinsicht wird die eingangs genannte Aufgabe dadurch gelöst, dass das Körperteil beim Andrücken in das Gehäuse hinein eindringt, wobei ein Entnahmebereich des Körperteils im Inneren des Gehäuses gegenüber einem flächigen Testfeld für eine Flüssigkeitsentnahme freigehalten wird.

Um die Benutzerfreundlichkeit weiter zu verbessern, ist es vorteilhaft, wenn im Gehäuseinneren auf einem Testband magazinierte Testfelder mit Körperflüssigkeit an dem exponierten Entnahmebereich des Körperteils beaufschlagt werden.

Zur Integration der einzelnen Analyseschritte wird im Gehäuseinneren eine Stecheinrichtung in den Entnahmebereich eingestochen, wobei unter dem erhöhten Innendruck aufgestaute Körperflüssigkeit aus der erzeugten Stichwunde austritt. Hierbei ist es günstig, wenn während einer Phase des Flüssigkeitsaustritts die Stecheinrichtung und das Testband von dem Entnahmebereich des Körperteils entfernt sind.

Vorteilhafterweise wird das vorzugsweise in einer Kassette aufgewickelte Testband vorgespult, so dass ein unverbrauchtes Testfeld in eine aktive Position bezüglich des Entnahmebereichs des Körperteils gelangt.

Eine weitere verfahrensmäßige Vereinfachung wird dadurch erreicht, dass ein Testfeld durch Querauslenkung des Testbands mit aus dem Entnahmebereich des Körperteils ausgetretener Körperflüssigkeit beaufschlagt wird.

Bevorzugt wird die Eindrücktiefe des Kompressionselements durch Anschlagmittel begrenzt.

Besonders günstig ist es auch, wenn das Kompressionselement über ein starres Trägerteil als Austauschteil an dem Gehäuse entnehmbar gehalten wird.

Im Folgenden wird die Erfindung anhand der in der Zeichnung schematisch dargestellten Ausführungsbeispiele näher erläutert. Es zeigen:
- Fig. 1: ein tragbares Analysegerät zur Blutzuckerbestimmung mit einer Vorrichtung zur Fingerpositionierung in perspektivischer Darstellung;
- Fig. 2 und 3: die aus dem Gerät entnehmbare Positioniervorrichtung in perspektivischer Darstellung und im Axialschnitt;
- Fig. 4: die Positioniervorrichtung mit angedrücktem Finger zur Beaufschlagung eines Testelements im Axialschnitt;
- Fig. 5: eine weitere Ausführungsform in einer Fig. 3 entsprechenden Darstellung; und
- Fig. 6a bis e: den Ablauf einer Blutentnahme/Analyse jeweils im Axialschnitt.

Die in der Zeichnung dargestellte Positioniervorrichtung 10 ist als leicht austauschbares Einsatzteil in das Gehäuse 12 eines tragbaren Blutzuckermessgeräts 14 einsetzbar, um eine Fingerbeere 16 eines Probanden im Geräteinneren 18 zur Blutentnahme und -analyse zu positionieren.

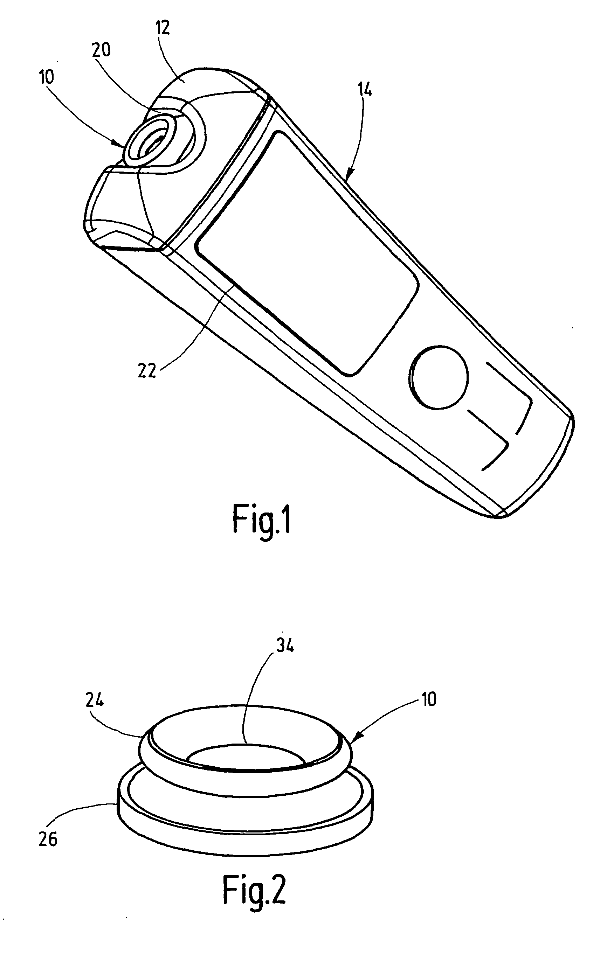

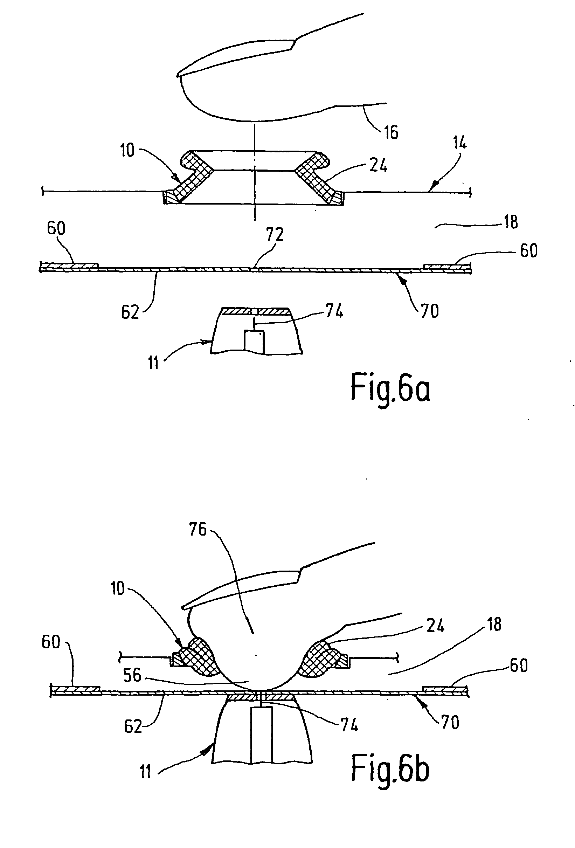

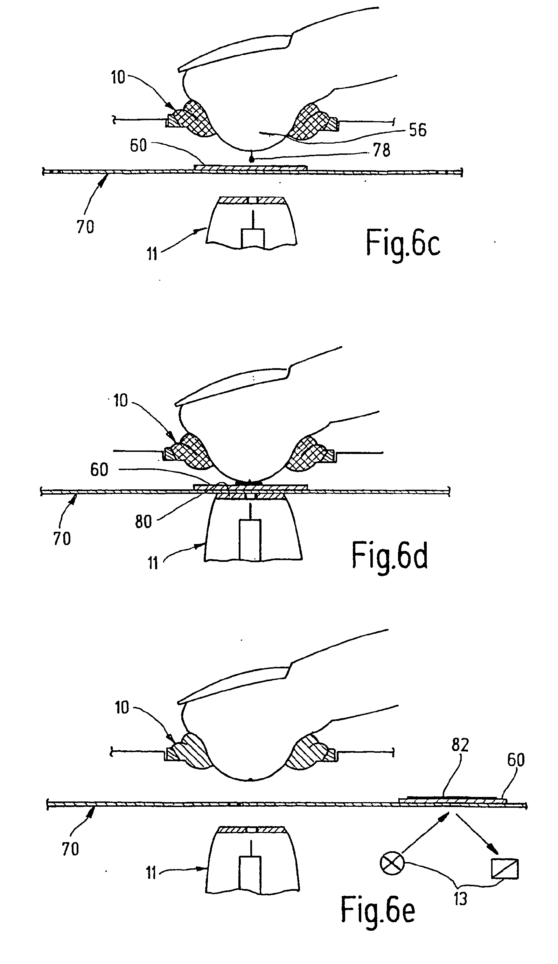

Zu diesem Zweck weist das in Fig. 1 gezeigte Handgerät 14 an einer Stirnseite eine Aufnahme 20 für die austauschbare Positioniervorrichtung 10 auf. Das Gerät umfasst als integriertes System eine geräteinterne Stecheinrichtung 11 zur Punktion der Fingerbeere und eine Analyseeinrichtung 13 zur Bestimmung des Zuckergehaltes des an der Einstichstelle entnommenen Blutes (Fig. 6). Das ermittelte Ergebnis lässt sich über eine Anzeigeeinheit 22 für den Benutzer darstellen.

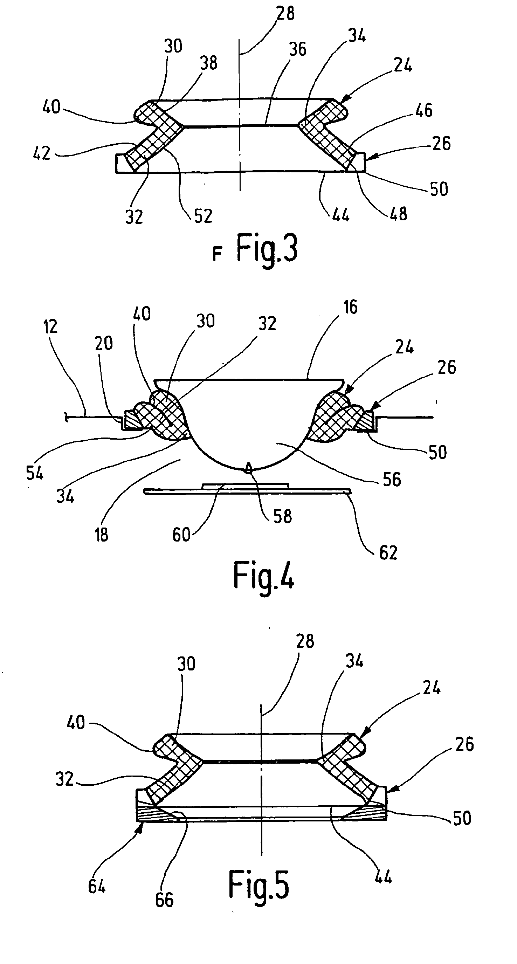

Wie am besten aus Fig. 2 und 3 ersichtlich, weist die Positioniervorrichtung 10 ein flexibles Kompressionselement 24 zum Andrücken der Fingerbeere 16 und ein formstabiles Trägerteil 26 zur Halterung des Kompressionselements 24 auf. Das im Querschnitt ringförmige Kompressionselement 24 besitzt zwei in Richtung der Andrückachse 28 konisch aufeinander zulaufende Teilbereiche 30, 32. Die Knickstelle zwischen diesen Teilbereichen 30, 32 bildet eine für den Eingriff der Fingerbeere 16 offene Eingriffspartie 34, die bei der Andrückbewegung ihre lichte Weite in dem Öffnungsbereich 36 verringert und so die freie Fingerkuppe unter Druckerhöhung einquetscht, um die Blutansammlung zu fördern.

Zur besseren Anpassung an die Fingerkontur weist der obere bzw. distale Teilbereich 30 eine konkav gewölbte innere Ringfläche 38 auf. Dadurch ergibt sich beim Andrücken des Fingers 16 zunächst ein kreisförmiger Kantenkontakt im Bereich der Eingriffspartie 34 mit hoher Flächenpressung für eine rutschsichere Positionierung.

Um die Andrückbewegung zuverlässig zu stoppen, ist an der freien Außenkante des distalen Teilbereichs 30 eine Anschlagwulst 40 angeformt, die sich zu dem proximalen Teilbereich 32 hin keilförmig verjüngt. Die Anschlagwulst 40 stützt sich in der Anschlagstellung an der Außenseite 42 des Teilbereichs 32 ab und verhindert somit ein weiteres Umklappen des Kompressionselements 24 in Andrückrichtung.

Das Trägerteil 26 begrenzt als Ringscheibe eine Durchtrittsöffnung 44 für das Kompressionselement 24. An seinem Öffnungsrand 46 ist das Trägerteil 26 über seine gesamte Höhe bzw. Wandstärke mittels Klebeverbindung fest mit dem Kompressionselement 24 verbunden. Für eine günstige Krafteinleitung ist die Verbindungsfläche 48 als Schräge unter einem spitzen Winkel von etwa 60° gegenüber der gehäuseseitigen Begrenzungsfläche 50 des Trägerteils angeordnet. Um eine große Eindrücktiefe zu erreichen, läuft die Innenfläche 52 des proximalen Teilbereichs 32 mit glatter Kontur knickfrei auf die gehäuseseitige Randkante des Öffnungsrandes 46 zu.

Die Kompressionseinheit 24 ist als flexibles Formteil aus Silikon gebildet, während das Trägerteil 26 aus Metall, vorzugsweise Aluminium besteht. Das Kompressionselement kann dabei im Spritzgussverfahren an das in ein Spritzgusswerkzeug eingelegte Trägerteil angeformt werden, wobei ein Primer an der Verbindungsfläche 48 die Festigkeit der Klebeverbindung erhöht. Alternativ ist auch eine Herstellung im Zweikomponenten-Spritzgussverfahren denkbar, wobei die Kompressionseinheit 24 aus einem thermoplastischen Elastomer und das Trägerteil 26 aus Kunststoff in einem Arbeitsgang als Verbundteil geformt werden.

Fig. 4 zeigt die End- bzw. Anschlagstellung der Kompressionseinheit 24 beim Andrücken des Fingers 16. Die keilförmige Anschlagwulst 40 füllt den außenseitigen Zwischenraum zwischen den Teilbereichen 30, 32 spaltfrei aus, so dass aufgrund der Inkompressibilität des Elastomermaterials auch bei geringem Reibungskoeffizient der Haut ein vollständiges Durchklappen verhindert wird.

Durch die tiefe Anbindung des proximalen Teilbereichs 32 an dem Öffnungsrand 46 wird die Knickstelle 54 entsprechend in Andrückrichtung nach unten verlagert. Die Eingriffspartie 34 ragt dadurch bis in das Innere 18 des Gehäuses 12 und jedenfalls unter die gehäuseseitige Begrenzungsfläche 50 des Trägerteils 26. Entsprechend wird ein Entnahmebereich 56 der Fingerbeere 16 im Gehäuseinneren 18 exponiert. Nach dem Einstich kann somit ein austretender Blutstropfen 58 frei durch ein flächiges Testfeld 60 abgenommen werden, ohne dass ein punktförmiger Zugriff erforderlich wäre. Diese Art der automatischen Blutgewinnung eignet sich besonders in Kombination mit einem Bandkassettensystem, mit dem eine Vielzahl von auf einem Trägerband 62 magazinierten Testfeldern 60 sukzessive in den Eingriffsbereich des Körperteils 16 transportiert werden können.

Bei der in Fig. 5 gezeigten Ausführungsform sind gleiche Teile mit den gleichen Bezugszeichen wie oben beschrieben versehen. Zusätzlich ist hier ein Widerlager 64 vorgesehen, das an der gehäuseseitigen Begrenzungsfläche 50 des Trägerteils 26 angebracht ist. Das Widerlager 64 ragt zur Begrenzung der Eindrücktiefe des Kompressionselements 24 in den Bereich der Öffnung 44 hinein. Dabei ist das Widerlager 64 in dem Öffnungsbereich zu einer freien Randkante hin nach unten abgeschrägt, so dass eine zu der Innenfläche 52 des Kompressionselements 24 gegenläufig abgeschrägte Anschlagfläche 66 für eine formschlüssige Abstützung entsteht. Dadurch wird eine zusätzliche Positioniergenauigkeit der Fingerkuppe 56 für die Blutentnahme im Geräteinneren erreicht.

In Fig. 6a bis e ist der Ablauf der Blutentnahme und Analyse in einzelnen Schritten dargestellt. In dem Gerät 14 ist die Positioniervorrichtung 10 als Austauschteil eingesetzt, während sich darunter im Geräteinneren 18 ein Testband 70 und die Stecheinrichtung 11 befinden. Das Testband 70 weist eine Vielzahl von Testfeldern 60 auf, die in Bandrichtung im Abstand voneinander auf dem Trägerband 62 angeordnet sind. Zwischen den Testfeldern 60 ist das Trägerband 62 mit Durchstechöffnungen 72 für das in der Stecheinrichtung 11 vorgespannte Stechelement 74 versehen. Das Testband 70 ist vorteilhafterweise in einer nicht gezeigten Kassette auf Spulen (Vorratsspule und Abfallspule) aufgewickelt.

Durch das Andrücken der Fingerkuppe 16 gegen das Kompressionselement 24 werden die Blutkapillaren in dem eingepressten Fingerbereich 76 abgeschnürt, während der Entnahmebereich 56 im Geräteinneren 18 für die Blutentnahme freigehalten wird (Fig. 6b). Zu diesem Zweck wird unter Vorschub des Stechelements 74 durch die Öffnung 72 hindurch eine Stichwunde erzeugt, aus der Blut 78 austreten kann. Um den Blutaustritt nicht zu behindern, befinden sich während dieser wenige Sekunden dauernden Phase die Stecheinrichtung 11 und das Testband 70 im Abstand von dem Entnahmebereich 56. Das Testband 70 wird hierbei durch Vorspulen so positioniert, dass ein unverbrauchtes Testfeld 60 dem Entnahmebereich 56 gegenüber liegt.

Gemäß Fig. 6d wird der ausgetretene Bluttropfen 78 direkt auf die Aufnahmefläche des Testfelds 60 übertragen, indem das Testband 70 durch eine distale Andrückfläche 80 der Stecheinrichtung 11 oder durch andere Mittel quer ausgelenkt wird. Aufgrund der Flächenausdehnung des Testelements 60 ist die Positionierung gegen die Stichwunde sehr variabel, während durch den direkten Zugriff beim Blutaufnehmen bzw. "Antupfen" ein Totvolumen durch Transportkapillaren oder dergleichen vermieden wird.

Wie in Fig. 6e gezeigt, wird das Testfeld 60 mit dem applizierten Blut 82 unter Bandvorschub in den Erfassungsbereich der optischen Analyseeinrichtung 13 transportiert. Auf dem Testfeld 60 befindet sich eine Testchemie, die mit einem Analyten, hier Glucose in dem Blut 82 beispielsweise unter Farbänderung reagiert. Dies lässt sich durch das transparente Trägerband hindurch reflektometrisch erfassen und für eine Anzeige und/oder Speicherung des Glucosemesswerts verarbeiten. Das verbrauchte Testfeld 60 wird auf der Abfallspule der Kassette hygienisch entsorgt, während ein frisches Testfeld für die nächste Messung wieder bereitgestellt wird, ohne dass der Benutzer in das Gerät eingreifen müsste.

## Patentansprüche

1. Analysegerät für Körperflüssigkeiten, insbesondere zur Blutzuckerbestimmung, mit einem Gehäuse (12) und einer Vorrichtung zur Positionierung eines Körperteils (16), insbesondere einer Fingerbeere, umfassend ein vorzugsweise ringförmiges flexibles Kompressionselement (24) zum Andrücken des Körperteils (16) unter Druckerhöhung und ein starres Trägerteil (26) zur Halterung des Kompressionselements (24) an dem Gehäuse (12), wobei im Gehäuseinneren (18) eine Analyseeinrichtung (13) zum Nachweis eines Analyten auf einem mit Körperflüssigkeit beaufschlagten Testfeld (60) angeordnet ist und das Kompressionselement (24) eine bei der Flüssigkeitsentnahme in das Gehäuse (12) hinein eingedrückte Eingriffspartie (34) für das Körperteil (16) aufweist, so dass ein Entnahmebereich (56) des Körperteils (16) im Inneren (18) des Gehäuses (12) gegenüber dem flächigen Testfeld (60) exponiert ist, wobei das Kompressionselement (24) zwei in Richtung einer Andrückachse (28) konisch aufeinander zulaufende Teilbereiche (30,32) aufweist, **gekennzeichnet dadurch**, dass der distale Teilbereich (30) eine beim Andrücken des Körperteils (16) außenseitig gegen den proximalen Teilbereich (32) anschlagende Anschlagwulst (40) zur Begrenzung der Eindrücktiefe aufweist.

2. Analysegerät nach Anspruch 1, **dadurch gekennzeichnet, dass** die Eingriffspartie (34) zumindest bis zu der Ebene einer gehäuseseitigen Begrenzung (50) des Trägerteils (26) eindrückbar ist.

3. Analysegerät nach Anspruch 1 oder 2, **dadurch gekennzeichnet,** dass das im Querschnitt ringförmige Kompressionselement (24) an einer freien Außenkante durch eine nach außen abstehende Anschlagwulst (40) verbreitert ist.

4. Analysegerät nach einem der Ansprüche 1 bis 3, **dadurch gekennzeichnet,** dass die Anschlagwulst (40) sich von der **freien Außenkante** des Kompressionselements (24) weg vorzugsweise keilförmig verjüngt.

5. Analysegerät nach einem der Ansprüche 1 bis 4, **dadurch gekennzeichnet,** dass das Trägerteil (26) eine Öffnung (44) zum Eindrücken des Kompressionselements (24) aufweist, und dass gehäuseseitig an dem Trägerteil (26) ein in den Öffnungsbereich ragendes Widerlager (64) zur Begrenzung der Eindrücktiefe des Kompressionselements (24) angebracht ist.

6. Analysegerät nach Anspruch 5, **dadurch gekennzeichnet,** dass das Widerlager (64) durch einen in die Öffnung (44) des Trägerteils (26) hinein sich verjüngenden Anschlagring gebildet ist.

7. Analysegerät nach einem der Ansprüche 1 bis 6, **dadurch gekennzeichnet,** dass das Kompressionselement (24) mit einem Öffnungsrand (46) des Trägerteils (26) fest verbunden ist, wobei die Verbindungsfläche (48) sich über die gesamte Höhe des Öffnungsrandes (46) erstreckt.

8. Analysegerät nach einem der Ansprüche 1 bis 7, **dadurch gekennzeichnet,** dass die Verbindungsfläche (48) zwischen Trägerteil (26) und Kompressionselement (24) als Schräge von einer gehäuseseitigen Begrenzungsfläche (50) des Trägerteils (26) abgewandt ist, und dass der Winkel zwischen der Schräge und der Begrenzungsfläche im Bereich von 50° bis 70°, vorzugsweise bei 60° liegt.

9. Analysegerät nach einem der Ansprüche 1 bis 8, **dadurch gekennzeichnet,** dass das Kompressionselement (24) in der Ausgangsstellung eine auf eine gehäuseseitige Randkante des Trägerteils (26) knickfrei zulaufende Innenfläche (52) aufweist.

10. Analysegerät nach einem der Ansprüche 1 bis 9, **dadurch gekennzeichnet,** dass die Eingriffspartie (34) durch die Innenkante einer konkav gewölbten Ringfläche (38) des Kompressionselements (24) gebildet ist.

11. Analysegerät nach einem der Ansprüche 1 bis 10, **dadurch gekennzeichnet,** dass das Kompressionselement (24) als Formteil, vorzugsweise als Spritzgussteil an dem vorgefertigten Trägerteil (26) angeformt ist.

12. Analysegerät nach einem der Ansprüche 1 bis 11, **dadurch gekennzeichnet,** dass das aus einem thermoplastischen Elastomer bestehende Kompressionselement (24) und das aus Kunststoff bestehende Trägerteil (26) als Zweikomponenten-Spritzgussteil ausgebildet sind.

13. Analysegerät nach einem der Ansprüche 1 bis 12, **dadurch gekennzeichnet,** dass das Kompressionselement (24) über eine insbesondere durch einen Primer gebildete Klebeverbindung an dem Trägerteil (26) befestigt ist.

14. Analysegerät nach einem der Ansprüche 1 bis 13, **dadurch gekennzeichnet,** dass das Kompressionselement (24) aus Silikon, Gummi oder Polyurethan besteht.

15. Analysegerät nach einem der Ansprüche 1 bis 14, **dadurch gekennzeichnet,** dass das Trägerteil (26) als formstabile Ringscheibe insbesondere aus Metall ausgebildet ist.

16. Analysegerät nach einem der Ansprüche 1 bis 15, **dadurch gekennzeichnet,** dass das Trägerteil (26) lösbar in eine Gehäuseaufnahme (20) einsetzbar ist.

17. Analysegerät nach einem der Ansprüche 1 bis 16, **dadurch gekennzeichnet,** dass die Gesamtbreite des Trägerteils (26) senkrecht zur Andrückrichtung (28) weniger als 25 mm, vorzugsweise weniger als 20 mm beträgt.

18. Analysegerät nach einem der Ansprüche 1 bis 17, **dadurch gekennzeichnet,** dass im Gehäuseinneren (18) ein Testband angeordnet ist, welches auf einem aufwickelbaren Trägerband (62) eine Vielzahl von Testfeldern (60) aufweist.

19. Analysegerät nach einem der Ansprüche 1 bis 18, **dadurch gekennzeichnet,** dass im Gehäuseinneren (18) eine Stecheinrichtung (11) zum Einstechen in den Entnahmebereich (56) des Körperteils (16) angeordnet ist.

20. Analysegerät nach einem der Ansprüche 1 bis 19, **dadurch gekennzeichnet,** dass im Gehäuseinneren (18) eine Analyseeinrichtung (13) zum Nachweis eines Analyten auf einem mit Körperflüssigkeit beaufschlagten Testfeld (60) angeordnet ist.

21. Analysegerät nach einem der Ansprüche 1 bis 20, **dadurch gekennzeichnet,** dass die aufeinanderfolgend auf dem vorzugsweise in einer Kassette befindlichen Testband angeordneten Testfelder (60) durch eine Bandtransportvorrichtung sukzessive in eine aktive Position bezüglich des Entnahmebereichs (56) des Körperteils (16), und/oder der Stecheinrichtung (11) und/oder der Analyseeinrichtung (13) bewegbar sind.

22. Analysegerät nach einem der Ansprüche 1 bis 21, **dadurch gekennzeichnet,** dass die Stecheinrichtung (11) eine distale Andrückfläche zum Andrücken eines Testfelds (60) gegen den Entnahmebereich (56) des Körperteils (16) aufweist.

23. Analysegerät nach einem der Ansprüche 1 bis 22, **dadurch gekennzeichnet,** dass die mit Körperflüssigkeit beaufschlagbare Aufnahmefläche des Testfelds (60) mehr als 2 x 5 mm², vorzugsweise 5 x 20 mm² beträgt.

24. Verfahren zur Analyse von Körperflüssigkeiten, insbesondere zur Blutzuckerbestimmung, bei dem ein Körperteil (16), insbesondere eine Fingerbeere, an einem Analysegerät (14) positioniert wird, wobei das Köperteil zur Erhöhung des Innendrucks an ein ringförmiges flexibles Kompressionselement (24) angedrückt wird, wobei im Gehäuseinneren (18) eine Analyseeinrichtung (13) zum Nachweis eines Analyten auf einem mit Körperflüssigkeit beaufschlagten Testfeld (60) angeordnet ist und wobei das Kompressionselement (24) eine in das Gehäuse (12) eindrückbare Eingriffspartie (34) für das Körperteil (16) aufweist und das Körperteil (16) beim Andrücken in das Gehäuse (12) hinein eindringt, so dass ein Entnahmebereich (56) des Körperteils (16) im Inneren (18) des Gehäuses (12) gegenüber dem flächigen Testfeld (60) für eine Flüssigkeitsentnahme freigehalten wird, wobei das Kompressionselement (24) zwei konisch aufeinander zulaufende Teilbereiche (30,32) aufweist, wobei der distale Teilbereich (30) eine Anschlagwulst (40) aufweist, und wobei die Eindrücktiefe durch die beim Andrücken des Körperteils (16) außenseitig gegen den proximalen Teilbereich (32) anschlagende Anschlagwulst (40) begrenzt wird.

25. Verfahren nach Anspruch 24, **dadurch gekennzeichnet,** dass im Gehäuseinneren (18) auf einem Testband magazinierte Testfelder (60) mit Körperflüssigkeit an dem exponierten Entnahmebereich (56) des Körperteils (16) beaufschlagt werden.

26. Verfahren Anspruch 24 oder 25, **dadurch gekennzeichnet,** dass im Gehäuseinneren (18) eine Stecheinrichtung (11) in den Entnahmebereich (56) eingestochen wird, wobei unter dem erhöhten Innendruck aufgestaute Körperflüssigkeit aus der erzeugten Stichwunde austritt.

27. Verfahren nach einem der Ansprüche 24 bis 26, **dadurch gekennzeichnet,** dass während einer Phase des Flüssigkeitsaustritts die Stecheinrichtung (11) und das Testband von dem Entnahmebereich (56) des Körperteils (16) entfernt sind.

28. Verfahren nach einem der Ansprüche 24 bis 27, **dadurch gekennzeichnet,** dass das vorzugsweise in einer Kassette aufgewickelte Testband vorgespult wird, so dass ein unverbrauchtes Testfeld (60) in eine aktive Position bezüglich des Entnahmebereichs (56) des Körperteils (16) gelangt.

29. Verfahren nach einem der Ansprüche 24 bis 28, **dadurch gekennzeichnet,** dass ein Testfeld (60) durch Querauslenkung des Testbands mit aus dem Entnahmebereich (56) des Körperteils (16) ausgetretener Körperflüssigkeit beaufschlagt wird.

30. Verfahren nach einem der Ansprüche 24 bis 29, **dadurch gekennzeichnet,** dass das Kompressionselement (24) über ein starres Trägerteil (26) als Austauschteil an dem Gehäuse (12) entnehmbar gehalten wird.

31. Verfahren nach einem der Ansprüche 24 bis 30, **dadurch gekennzeichnet,** dass die Eingriffspartie (34) zumindest bis zu der Ebene einer gehäuseseitigen Begrenzung (50) des Trägerteils (26) eingedrückt wird.

## Claims

1. Analytical instrument for body fluids, in particular for determining blood sugar, comprising a housing (12) and a device for positioning a body part (16), in particular a finger pad, comprising a preferably circular flexible compression element (24) for pressing against the body part (16) while increasing the pressure and a rigid support part (26) to hold the compression element (24) on the housing (12), wherein an analytical device (13) for detecting an analyte on a test field (60) to which body fluid has been applied is arranged in the interior of the housing (18) and the compression element (24) has an engaging member (34) for the body part (16) which is pressed into the housing (12) during liquid withdrawal such that a withdrawal region (56) of the body part (16) is exposed in the interior (18) of the housing (12) opposite to a flat test field (60), wherein the compression element (24) has two subregions (30, 32) which converge in a conical manner to each other in direction of a pressure axis (28), **characterized in that** the distal subregion (30) has a stop bead (40) which abuts against the outside of the proximal subregion (30) in order to limit the impression depth when the body part (16) is pressed on.

2. Analytical instrument according to claim 1, **characterized in that** the engaging member (34) can be pressed in at least up to the plane of a boundary (50) of the support part (26) on the side of the housing.

3. Analytical instrument according to claim 1 or 2, **characterized in that** the compression element (24) with a circular cross-section has a free outer edge that is widened by an outwardly projecting stop bead (40).

4. Analytical instrument according to one of the claims 1 to 3, **characterized in that** the stop bead (40) tapers preferably in a wedge-like manner away from the free outer edge of the compression element (24).

5. Analytical instrument according to one of the claims 1 to 4, **characterized in that** the support part (26) has an opening (44) to press in the compression element (24) and that an abutment (64) extending into the area of the opening region is attached to the housing side of the support part (26) in order to limit the depth of impression of the compression element (24).

6. Analytical instrument according to claim 5, **characterized in that** the abutment (64) is formed by a stop ring which tapers into the opening (44) of the support part (26).

7. Analytical instrument according to one of the claims 1 to 6, **characterized in that** the compression element (24) is permanently connected to a rim of the opening (46) in the support part (26) wherein the connecting area (48) extends over the entire height of the rim of the opening (46).

8. Analytical instrument according to one of the claims 1 to 7, **characterized in that** the area (48) connecting the support part (26) and compression element (24) is inclined away from a boundary surface (50) of the support part (26) on the housing where the angle between the incline and the boundary surface is in the range of 50° to 70°, preferably 60°.

9. Analytical instrument according to one of the claims 1 to 8, **characterized in that** the compression element (24) in the initial position has an inner surface (52) which tapers in a kink-free manner towards a lateral edge of the support part (26) on side of the housing.

10. Analytical instrument according to one of the claims 1 to 9, **characterized in that** the engaging member (34) is formed by the inner edge of a concavely curved ring surface (38) of the compression element (24).

11. Analytical instrument according to one of the claims 1 to 10, **characterized in that** the compression element (24) is moulded as a moulded part, preferably as an injection moulded part onto the prefabricated support part (26).

12. Analytical instrument according to one of the claims 1 to 11, **characterized in that** the compression element (24) consisting of a thermoplastic elastomer and the support part (26) consisting of plastic are formed as a two component injection moulded part.

13. Analytical instrument according to one of the claims 1 to 12, **characterized in that** the compression element (24) can be attached to the support part (26) by an adhesive bond and in particular by an adhesive bond formed by a primer.

14. Analytical instrument according to one of the claims 1 to 13, **characterized in that** the compression element (24) is composed of silicon, rubber or polyurethane.

15. Analytical instrument according to one of the claims 1 to 14, **characterized in that** the support part (26) is constructed as a dimensionally stable ring washer and especially one made of metal.

16. Analytical instrument according to one of the claims 1 to 15, **characterized in that** the support part (26) can be inserted into a housing receiving member (20) in a detachable manner.

17. Analytical instrument according to one of the claims 1 to 16, **characterized in that** the total width of the support part (26) perpendicular to the pressing direction (28) is less than 25 mm, preferably less than 20 mm.

18. Analytical instrument according to one of the claims 1 to 17, **characterized in that** a test tape is arranged in the interior of the housing (18) which has a plurality of test fields (60) on a windable support tape (62).

19. Analytical instrument according to one of the claims 1 to 18, **characterized in that** a lancing device (11) for insertion into the withdrawal region (56) of the body part (16) is arranged in the interior of the housing (18).

20. Analytical instrument according to one of the claims 1 to 19, **characterized in that** an analytical device (13) for detecting an analyte on a test field (60) to which body fluid has been applied is arranged in the interior of the housing (18).

21. Analytical instrument according to one of the claims 1 to 20, **characterized in that** the test fields (60) arranged consecutively on the test tape that is preferably located in a cassette can be moved successively into an active position with regard to the withdrawal region (56) of the body part (16), and/or the lancing device (11) and/or the analytical device (13) by means of a tape transport device.

22. Analytical instrument according to one of the claims 1 to 21, **characterized in that** the lancing device (11) has a distal pressing face to press a test field (60) against the withdrawal region (56) of the body part (16).

23. Analytical instrument according to one of the claims 1 to 22, **characterized in that** the receiving area of the test field (60) to which body fluid can be applied is more than 2 x 5 mm² and preferably 5 x 20 mm².

24. Method for analysing body fluids, in particular for determining blood sugar, in which a body part (16), in particular a finger pad, is positioned on an analyser (14), wherein the body part is pressed against a circular flexible compression element (24) in order to increase the internal pressure, wherein an analytical device (13) for detecting an analyte on a test field (60) to which body fluid has been applied is arranged in the interior of the housing (18) and wherein the compression element (24) has an engaging member (34) for the body part (16) which can be pressed into the housing (12) and the body part (16) penetrates into the housing (12) when it is pressed down, such that a withdrawal region (56) of the body part (16) is kept clear in the interior (18) of the housing (12) opposite to the flat test field (60) in order to remove liquid, wherein the compression element (24) has two subregions (30, 32) which converge in a conical manner towards each other, wherein the distal subregion (30) has a stop bead (40), and wherein the impression depth is limited by means of the stop bead (40) which abuts against the outside the proximal subregion (30) when the body part (16) is pressed on.

25. Method according to claim 24, **characterized in that** body fluid on the exposed withdrawal region (56) of the body part (16) is applied to test fields (60) stored on a test tape in the interior of the housing (18).

26. Method according to claim 24 or 25, **characterized in that** a lancing device (11) is inserted into the withdrawal region (56) in the interior of the housing (18) whereby body fluid dammed up under the increased inner pressure emerges from the generated puncture wound.

27. Method according to one of the claims 24 to 26, **characterized in that** the lancing device (11) and the test tape are removed from the withdrawal region (56) of the body part (16) during a phase of liquid efflux.

28. Method according to one of the claims 24 to 27, **characterized in that** a test tape preferably wound up in a cassette is advantageously wound on such that an unused test field (60) is moved to an active position relative to the withdrawal region (56) of the body part (16).

29. Method according to one of the claims 24 to 28, **characterized in that** a body fluid that has escaped from the withdrawal region (56) of the body part (16) is applied to a test field (60) by a lateral excursion of the test tape.

30. Method according to one of the claims 24 to 29, **characterized in that** the compression element (24) is held on the housing (12) as a replaceable part in a removable manner by means of a rigid support part (26).

31. Method according to one of the claims 24 to 30 **characterized in that** the engaging member (34) can be pressed in at least up to the plane of a boundary (50) of the support part (26) on the side of the housing.

## Revendications

1. Appareil d'analyse pour liquides corporels, notamment pour déterminer le taux de glycémie, avec un boîtier (12) et un dispositif pour positionner une partie du corps (16), en particulier la pulpe du doigt, comprenant un élément de compression (24) flexible, de préférence de forme annulaire, permettant d'appliquer la partie du corps (16) en augmentant la pression et un élément de support (26) rigide permettant de fixer l'élément de compression (24) sur le boîtier (12), un dispositif d'analyse (13) pour la détection d'un analyte sur une zone de test (60) recevant du liquide corporel étant placé à l'intérieur (18) du boîtier et l'élément de compression (24) comportant une partie d'engagement (34) pour la partie du corps (16), qui est enfoncée dans le boîtier (12) lors du prélèvement de liquide, de sorte qu'une zone de prélèvement (56) de la partie du corps (16) est exposée à l'intérieur (18) du boîtier (12) vis-à-vis de la zone de test (60) surfacique, l'élément de compression (24) ayant deux portions (30, 32) s'étendant de manière conique l'une vers l'autre en direction d'un axe d'application (28), **caractérisé en ce que** la portion distale (30) comporte un bourrelet d'arrêt (40) butant extérieurement contre la portion proximale (32) en appliquant la partie du corps (16) pour limiter la profondeur d'enfoncement.

2. Appareil d'analyse selon la revendication 1, **caractérisé en ce que** la partie d'engagement (34) peut s'enfoncer au moins jusqu'au niveau d'une délimitation (50) côté boîtier de l'élément de support (26).

3. Appareil d'analyse selon la revendication 1 ou 2, **caractérisé en ce que** l'élément de compression (24) de section annulaire est élargi sur un bord extérieur libre par un bourrelet d'arrêt (40) saillant vers l'extérieur.

4. Appareil d'analyse selon l'une des revendications 1 à 3, **caractérisé en ce que** le bourrelet d'arrêt (40) se rétrécit, de préférence en forme de coin, depuis le bord extérieur libre de l'élément de compression (24).

5. Appareil d'analyse selon l'une des revendications 1 à 4, **caractérisé en ce que** l'élément de support (26) comporte une ouverture (44) permettant d'enfoncer l'élément de compression (24), et **en ce que** côté boîtier un contre-appui (64) pénétrant dans la zone d'ouverture est monté sur l'élément de support (26) pour limiter la profondeur d'enfoncement de l'élément de compression (24).

6. Appareil d'analyse selon la revendication 5, **caractérisé en ce que** le contre-appui (64) est formé par une bague de butée se rétrécissant vers l'intérieur de l'ouverture (44) de l'élément de support (26).

7. Appareil d'analyse selon l'une des revendications 1 à 6, **caractérisé en ce que** l'élément de compression (24) est relié solidement à un bord d'ouverture (46) de l'élément de support (26), la surface de liaison (48) s'étendant sur toute la hauteur du bord d'ouverture (46).

8. Appareil d'analyse selon l'une des revendications 1 à 7, **caractérisé en ce que** la surface de liaison (48), de forme oblique, entre élément de support (26) et élément de compression (24) est opposée à une surface de délimitation (50) de l'élément de support (26), et **en ce que** l'angle entre la surface oblique et la surface de délimitation se situe dans la plage allant de 50° à 70°, de préférence à 60°.

9. Appareil d'analyse selon l'une des revendications 1 à 8, **caractérisé en ce que** l'élément de compression (24) présente en position initiale une surface intérieure (52) arrivant sans pli sur une arête de bord côté boîtier de l'élément de support (26).

10. Appareil d'analyse selon l'une des revendications 1 à 9, **caractérisé en ce que** la partie d'engagement (34) est formée par le bord intérieur d'une surface annulaire (38) à courbure concave de l'élément de compression (24).

11. Appareil d'analyse selon l'une des revendications 1 à 10, **caractérisé en ce que** l'élément de compression (24) est formé comme pièce moulée, de préférence comme pièce moulée par injection, sur l'élément de support (26) préfabriqué.

12. Appareil d'analyse selon l'une des revendications 1 à 11, **caractérisé en ce que** l'élément de compression (24) constitué d'un élastomère thermoplastique et l'élément de support (26) constitué de matière plastique sont réalisés sous forme de pièce moulée par injection à deux composants.

13. Appareil d'analyse selon l'une des revendications 1 à 12, **caractérisé en ce que** l'élément de compression (24) est fixé sur l'élément de support (26) au moyen d'un assemblage collé, constitué notamment par un primaire.

14. Appareil d'analyse selon l'une des revendications 1 à 13, **caractérisé en ce que** l'élément de compression (24) est constitué de silicone, de caoutchouc ou de polyuréthane.

15. Appareil d'analyse selon l'une des revendications 1 à 14, **caractérisé en ce que** l'élément de support (26) est réalisé sous forme de disque annulaire indéformable notamment en métal.

16. Appareil d'analyse selon l'une des revendications 1 à 15, **caractérisé en ce que** l'élément de support (26) peut être mis en place de manière amovible dans un logement (20) de boîtier.

17. Appareil d'analyse selon l'une des revendications 1 à 16, **caractérisé en ce que** la largeur totale de l'élément de support (26), perpendiculaire à la direction d'application (28), est inférieure à 25 mm, de préférence inférieure à 20 mm.

18. Appareil d'analyse selon l'une des revendications 1 à 17, **caractérisé en ce qu**'à l'intérieur (18) du boîtier est placée une bande de test présentant sur une bande support (62) enroulable une pluralité de zones de test (60).

19. Appareil d'analyse selon l'une des revendications 1 à 18, **caractérisé en ce qu**'à l'intérieur (18) du boîtier est placé un dispositif de piqûre (11) pour piquer la zone de prélèvement (56) de la partie du corps (16).

20. Appareil d'analyse selon l'une des revendications 1 à 19, **caractérisé en ce** qu'à l'intérieur (18) du boîtier est placé un dispositif d'analyse (13) pour la détection d'un analyte sur une zone de test (60) recevant du liquide corporel.

21. Appareil d'analyse selon l'une des revendications 1 à 20, **caractérisé en ce que** les zones de test (60), placées les unes à la suite des autres sur la bande de test se trouvant de préférence dans une cassette, peuvent être successivement déplacées par un dispositif de transport de bande dans une position active par rapport à la zone de prélèvement (56) de la partie du corps (16) et/ou par rapport au dispositif de piqûre (11) et/ou par rapport au dispositif d'analyse (13).

22. Appareil d'analyse selon l'une des revendications 1 à 21, **caractérisé en ce que** le dispositif de piqûre (11) comporte une surface d'application distale permettant d'appliquer une zone de test (60) contre la zone de prélèvement (56) de la partie du corps (16).

23. Appareil d'analyse selon l'une des revendications 1 à 22, **caractérisé en ce que** la surface réceptrice de liquide corporel de la zone de test (60) est supérieure à 2 x 5 mm², de préférence égale à 5 x 20 mm².

24. Procédé d'analyse de liquides corporels, notamment pour déterminer le taux de glycémie, dans lequel une partie du corps (16), en particulier la pulpe du doigt, est positionnée sur un appareil d'analyse (14), laquelle partie du corps, de manière à augmenter la pression interne, est appliquée contre un élément de compression (24) flexible de forme annulaire, un dispositif d'analyse (13) pour la détection d'un analyte sur une zone de test (60) recevant du liquide corporel étant placé à l'intérieur (18) du boîtier et l'élément de compression (24) comportant une partie d'engagement (34) enfonçable dans le boîtier (12) pour la partie du corps (16), laquelle partie du corps (16) pénètre dans le boîtier (12) pendant l'application, de sorte qu'une zone de prélèvement (56) de la partie du corps (16) reste dégagée à l'intérieur (18) du boîtier (12) vis-à-vis de la zone de test (60) surfacique pour un prélèvement de liquide, l'élément de compression (24) ayant deux portions (30, 32) s'étendant de manière conique l'une vers l'autre, la portion distale (30) comportant un bourrelet d'arrêt (40), lequel bourrelet d'arrêt (40), en butant extérieurement contre la portion proximale (32) lorsqu'on applique la partie du corps (16), limite la profondeur d'enfoncement.

25. Procédé selon la revendication 24, **caractérisé en ce qu'**à l'intérieur (18) du boîtier, les zones de test (60) chargées sur une bande de test reçoivent du liquide corporel au niveau de la zone de prélèvement exposée (56) de la partie du corps (16).

26. Procédé selon la revendication 24 ou 25, **caractérisé en ce qu'**à l'intérieur (18) du boîtier, un dispositif de piqûre (11) est introduit dans la zone de prélèvement (56) et, sous l'effet de la pression interne augmentée, du liquide corporel accumulé s'écoule de l'incision réalisée.

27. Procédé selon l'une des revendications 24 à 26, **caractérisé en ce que** pendant une phase d'écoulement de liquide, le dispositif de piqûre (11) et la bande de test sont éloignés de la zone de prélèvement (56) de la partie du corps (16).

28. Procédé selon l'une des revendications 24 à 27, **caractérisé en ce que** la bande de test, enroulée de préférence dans une cassette, est avancée de manière à placer une zone de test (60) non utilisée dans une position active par rapport à la zone de prélèvement (56) de la partie du corps (16).

29. Procédé selon l'une des revendications 24 à 28, **caractérisé en ce qu**'une zone de test (60) reçoit du liquide corporel s'écoulant de la zone de prélèvement (56) de la partie du corps (16) suite à un débattement transversal de la bande de test.

30. Procédé selon l'une des revendications 24 à 29, **caractérisé en ce que** l'élément de compression (24) est fixé comme pièce de rechange de manière amovible sur le boîtier (12), au moyen d'un élément de support (26) rigide.

31. Procédé selon l'une des revendications 24 à 30, **caractérisé en ce que** la partie d'engagement (34) est enfoncée au moins jusqu'au niveau d'une délimitation (50) côté boîtier de l'élément de support (26).
